# EUROPEAN PATENT APPLICATION

(11) **EP 4 649 895 A1**
(43) Date of publication of application: **19.11.2025**
(21) Application number: 25176777.8
(22) Date of filing: 15.05.2025
(51) Int. Cl.: A61B 17/04

(54) **INSTRUMENTATION FOR INSERTION OF A SUTURE ANCHOR**

(30) Priority: 15.05.2024 US 202463647872 P
(71) Applicant: Stryker Corporation, Portage, MI 49002 (US)
(72) Inventor: CARDENAS, Ruben, Portage, 49002 (US); SMITH, Conrad, Portage, 49002 (US); LOPES, Edson Falcao, Portage, 49002 (US)
(74) Representative: V.O.

(57) **Abstract**

An insertion tool for inserting a suture anchor into bone includes three concentric shafts coupled to a handle. An intermediate shaft is rotatable relative to the handle to rotate the anchor body. An inner shaft within the intermediate shaft has a distal end for coupling to an eyelet positionable distally of the anchor body. An outer shaft disposed about the intermediate shaft translates distally to advance the anchor body towards the eyelet. A retention suture and at least one repair suture may be coupled to the eyelet such that their free ends are coupled to the handle. The free ends of the retention suture extend within the intermediate shaft and through a space between a ring gear and a spur gear of the handle. During operation, the distance between the handle and the distal end of the inner shaft remains constant so as to maintain tension in the repair suture.

## Description

### BACKGROUND OF THE INVENTION

There are many medical procedures where a surgeon needs to attach soft tissue to bone. The soft tissue can be, for example, ligaments, tendons, or other connective tissue. One very common example of this is rotator cuff repair where a portion or all of the rotator cuff is torn or detached from the humerus. When the rotator cuff tears from the humerus, the result is pain and loss of function. When a patient presents with a significant rotator cuff tear, surgical repair is performed. The goal of surgical repair of the rotator cuff is to secure the tendon to the bone in a stable manner so that the tendon can reattach to the bone and can heal. If the tendon is not stable and oscillation or micro-motion between the tendon and bone develops, the healing process will be interrupted. In this situation, it is less likely that the tendon will heal properly to the bone, resulting in a re-tear. Thus, the more stable the repair, the more successfully the tendon will heal to the bone.

One common way of attaching soft tissue to bone is via the use of one or more suture anchors. Suture anchors are components that are implanted and affixed within a hole in bone, such that the suture anchor serves as a point of fixation to the bone for one or more sutures or other similar flexible components, which can then be used to directly or indirectly attach to the soft tissue. One form of suture anchor involves two components, including an anchor body configured to secure itself within the bone hole, and the other component is a separate eyelet structure, which includes an opening through it that is configured to have one or more sutures pass through it so as to secure the suture(s) to the anchor. The eyelet may be secured to the insertion instrument by, for example, a friction fit with the distal end of the inner shaft-like component and/or by a suture (known as a "retention suture") that is secured to some portion of the insertion instrument. Such retention suture may pass through the opening of the eyelet along with one or more "repair sutures," which repair sutures are subsequently used for securing the soft tissue.

Although many improvements have been made in the field of suture anchors and the instrumentation for inserting them, further improvement would be desirable.

### BRIEF SUMMARY OF THE INVENTION

Some aspects of the present invention provide an insertion tool for inserting a suture anchor into bone. One such insertion tool desirably includes a handle, an intermediate shaft, an inner shaft, and an outer shaft. The intermediate shaft, the inner shaft, and the outer shaft may be concentrically disposed with respect to one another along a longitudinal axis, with the inner shaft being concentrically disposed within the intermediate shaft and the outer shaft being concentrically disposed about the intermediate shaft. A proximal end of the intermediate shaft may be rotatably coupled to the handle, and a distal end of the intermediate shaft may be configured to couple to an anchor body of the suture anchor. The intermediate shaft may be rotatable relative to the handle so as to rotate the anchor body relative to the handle. The inner shaft may have a distal end configured to couple to an eyelet positionable distally of and separate from the anchor body. The outer shaft may be configured to translate distally relative to the handle so as to advance the anchor body distally towards the eyelet.

In some of the above aspects of the insertion tool, rotation of the intermediate shaft relative to the handle and distal translation of the outer shaft relative to the handle may both be driven by a rotatable knob coupled to the handle. In some of such aspects, a proximal end of the outer shaft may be movably coupled to the handle and to the rotatable knob via at least one spline aligned along the longitudinal axis and via a lead screw thread. Due to such movable coupling, rotation of the rotatable knob preferably drives distal translation of the outer shaft relative to the handle.

In some other of the above aspects of the insertion tool, the handle may have a rotatable knob coupled thereto for driving rotation of the intermediate shaft relative to the handle via a ring gear engaged with a spur gear. The ring gear and the spur gear may define a space between them such that a retention suture coupled to the eyelet and extending within the intermediate shaft can pass through the space and out of the handle. In some of such aspects, the ring gear and the spur gear may define a gear ratio therebetween such that the anchor body rotates at a different rate than the rotatable knob.

In some aspects of the insertion tool, the inner shaft may be rotationally fixed relative to the handle. In other aspects of the insertion tool, the distal end of the intermediate shaft may have a keyed outer profile complementary to an inner profile of a passage within the anchor body. In yet other aspects of the insertion tool, the handle may include at least one cleat along an outer surface of the handle. Such cleat may be configured to secure a free end of a repair suture extending proximally from the eyelet.

In further aspects of the invention, a system may be provided for inserting a suture anchor into bone. One such system may include an anchor body, an eyelet, and an insertion tool in accordance with any of the above aspects of the invention. In at least some of such aspects of the system, a repair suture and a retention suture may also be provided. The repair suture may be for securing tissue and may be configured to extend through the eyelet. The retention suture may be for helping to secure the eyelet in a position coupled to the distal end of the inner shaft. The retention suture may be configured to extend within the intermediate shaft of the insertion tool from the eyelet to the handle.

In some of the above aspects of the system, the handle may have a rotatable knob coupled thereto for driving rotation of the intermediate shaft relative to the handle and for driving distal translation of the outer shaft relative to the handle. A proximal end of the outer shaft may be movably coupled to the handle and to the rotatable knob via at least one spline aligned along the longitudinal axis and via a lead screw thread. In at least some of such aspects, a thread pitch of the lead screw thread may match a thread pitch of a thread along an outer surface of the anchor body. As a result of such matching, the outer shaft will desirably translate distally relative to the handle so as to match the distal threaded advancement of the anchor body into bone.

In some of the above aspects of the system, the eyelet is coupled to a distal end of the inner shaft of the insertion tool, and the system is configured for distally advancing the externally threaded anchor body of the suture anchor into a bore within the bone by rotating the anchor body about the axis while pushing the anchor body distally into the bore along the axis. The rotating of the anchor body about the axis can include rotating the intermediate shaft of the insertion tool about the axis. The intermediate shaft can have the distal end coupled to the anchor body. The pushing of the anchor body distally into the bore can include translating the outer shaft of the insertion tool distally relative to the intermediate shaft.

In some of the above aspects, the repair suture extends through the eyelet and has a free end secured to the cleat of the handle of the insertion tool. The system can be configured to maintain tension in the repair suture between the eyelet and the cleat while distally advancing the anchor, such as by maintaining a distance between the handle and the distal end of the inner shaft while the anchor body is distally advanced.

Other aspects of the present invention provide a method for inserting a suture anchor into bone. A method in accordance with that aspect of the invention desirably includes distally advancing an eyelet into a bore within a bone, and distally advancing an externally threaded anchor body of a suture anchor into the bore within the bone. According to such method, the eyelet may be coupled to a distal end of the inner shaft of an insertion tool during the distal advancement of the eyelet into the bore. Also, according to such method, the distal advancement of the anchor body may be induced by rotating the anchor body about an axis while pushing the anchor body distally into the bore along the axis. Such rotation of the anchor body about the axis may include rotating an intermediate shaft of the insertion tool about the axis, which intermediate shaft has a distal end coupled to the anchor body. The pushing of the anchor body distally into the bore may include translating an outer shaft of the insertion tool distally relative to the intermediate shaft. In accordance with the above method, the intermediate shaft may be concentrically disposed about the inner shaft and the outer shaft may be concentrically disposed about the intermediate shaft.

In some of the above aspects of the method, a repair suture may extend through the eyelet and may have a free end secured to a cleat of a handle of the insertion tool. Furthermore, distally advancing the anchor body into the bore may include maintaining tension in the repair suture between the eyelet and the cleat. In at least some of such aspects, the maintaining of the tension in the repair suture may include maintaining a distance between the handle and the distal end of the inner shaft while the anchor body is distally advanced into the bore.

In some aspects of the method, the eyelet preferably does not rotate during the distal advancement of the anchor body into the bore. In other aspects of the method, the rotating of the intermediate shaft and the translating of the outer shaft distally may both be driven by rotating a knob coupled to a handle of the insertion tool. In yet other aspects of the method, the rotating of the intermediate shaft may be driven by rotating a knob coupled to a handle of the insertion tool, which knob drives rotation of the intermediate shaft via a ring gear engaged with a spur gear. In such aspects, a free end of a retention suture may extend out of the handle during the distal advancement of the anchor body into the bore. Such retention suture may extend from the eyelet to the free end by passing within the intermediate shaft of the insertion tool and through a space defined between the ring gear and the spur gear.

In some aspects of the method, distally advancing the eyelet into the bore may include creating the bore within the bone by distally advancing the eyelet. Alternatively, in other aspects of the method, the bore within the bone may have been formed before the step of distally advancing the eyelet into the bore.

### BRIEF DESCRIPTION OF THE DRAWINGS

FIG. 1 is a perspective view of an insertion tool and suture anchor of a suture anchor system in accordance with an embodiment of the present invention.
FIG. 2 is a perspective view of an anchor body and eyelet of a suture anchor of the suture anchor system of FIG. 1.
FIG. 3A is a perspective view of the distal end of an insertion tool of the suture anchor system of FIG. 1.
FIG. 3B is a perspective view of the anchor body and eyelet of the suture anchor of FIG. 2 positioned on the distal end of the insertion tool of the embodiment of FIG. 1.
FIG. 4 is a cross sectional view of the handle of the insertion tool of the embodiment of FIG. 1.
FIG. 5 is a perspective view of a drive shaft and lead screw member shown isolated from the handle of the insertion tool of the embodiment of FIG. 1.
FIG. 6 is a perspective view of an assembly of the components of the handle of the insertion tool of the embodiment of FIG. 1, with the handle body being omitted.
FIG. 7 is a side perspective view of a portion of an assembly of some of the components of the insertion tool of FIG. 1, focusing on the suture guide, the ring gear of the drive shaft, and the proximal portion of the lead screw member.
FIG. 8 is a side view of the portion of the assembly of components of FIG. 7, except that all but the pins of the suture guide is omitted so as to show a path of the retention suture.
FIG. 9 is a perspective view of the portion of the assembly of components of FIG. 7, viewed along a distal direction.
FIG. 10 is a cross sectional view of the handle of the insertion instrument of FIG. 1, taken orthogonal to the longitudinal axis so as to show the interaction of the ring gear and the spur gear.
FIG. 11 is a perspective view of the handle of the insertion tool of the embodiment of FIG. 1.
FIG. 12 is a perspective view of a handle of an alternative embodiment of an insertion tool, in which part of the handle body has been cut away.
FIG. 13 is a cross-sectional view of the handle of FIG. 12, taken along the longitudinal axis.

### DETAILED DESCRIPTION

As used herein, the word "proximal" or "proximally" refers to a direction closer to or towards the user (*e.g*., surgeon, medical staff, and the like), whereas the word "distal" or "distally" refers to a direction further from or away from the operator.

As used herein, the term "filament" and "suture" and like terms are inclusive of single or multiple strands, threads, fibers, tapes, strings, or wires, in which such terms preferably refer to a thread-like suture material, and in particular a braided suture, having a hollow core along at least a portion of its length. A filament or suture may be constructed from homogenous or heterogeneous materials such as, but not limited to, polyester, polyethylene (including ultra-high molecular weight polyethylene (UHMWPE)), polytetrafluorethylene (including expanded polytetrafluorethylene), nylon, polypropylene, aramids (such as Kevlar-based materials), polydioxanone, polygycolic acid or other absorbable material, liquid crystal polymer (LCP), organic material (silk, animal tendon, or the like), metallic wire, or any combination of these materials. For example, the suture could constitute two portions of a suture that is passed through tissue or graft material whereby the two portions, or end portions, are brought to the implant. Alternatively, the filament could constitute multiple lengths of separate sutures which together are engaged by the implant.

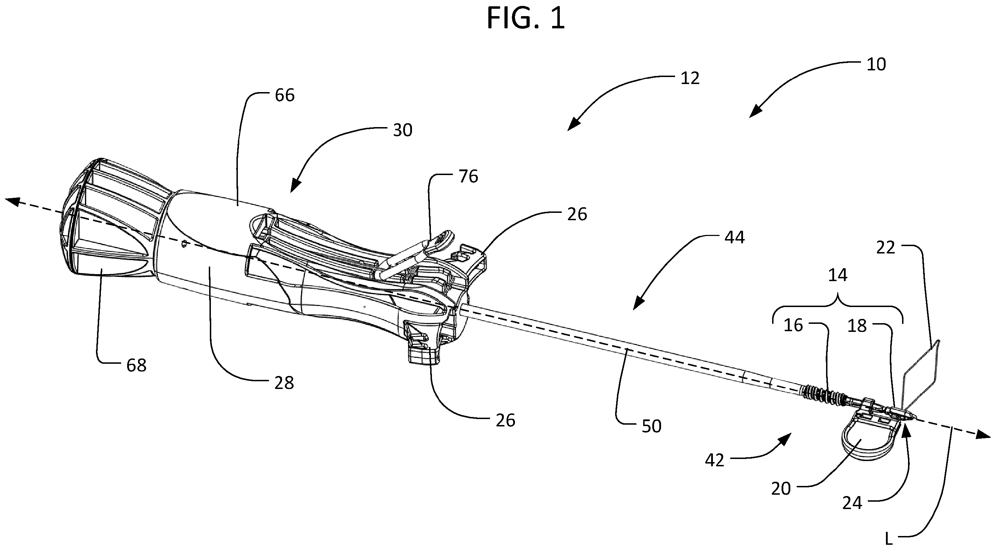

An embodiment of a suture anchor system 10 in accordance with an embodiment of the present invention is illustrated in FIG. 1. The suture anchor system 10 includes an insertion tool 12 having a suture anchor 14 positioned at a distal end thereof. The suture anchor 14 is a two-part component including an anchor body 16 and a separate eyelet 18 positioned distally of the anchor body 16. FIG. 1 also illustrates a pull tab 20 connected to a loop 22 of wire (*e.g*., nitinol wire) extending through an opening 24 in the eyelet 18 so that one or more repair sutures (not shown) can be easily pulled through the opening 24 in the eyelet 18. Before implanting the suture anchor, the free ends of such repair sutures may be pulled proximally and secured to one or more cleats 26 projecting outwardly along an outer surface 28 of a handle body 66 of a handle 30 of the insertion tool 12.

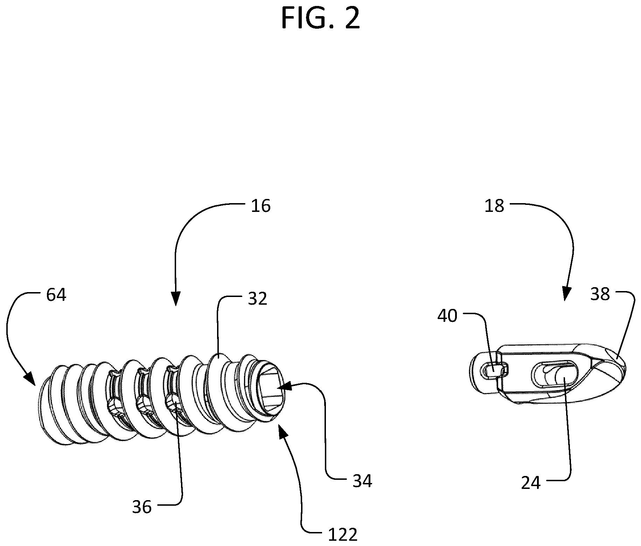

FIG. 2 illustrates the two components of the suture anchor 14 noted above (*i.e.,* the anchor body 16 and the eyelet 18). Components suitable for use as the anchor body 16 and eyelet 18 include any of the different embodiments of fixation members and eyelets, respectively, as disclosed in U.S. Patent No. 10,722,343, the entire disclosure of which is incorporated by reference herein. As shown in FIG. 2, the anchor body 16 is a cannulated, generally cylindrical body having one or more screw threads 32 along the outside. Such screw threads 32 are configured to form an interference fit with a hole in bone in order to anchor the anchor body 16 within the hole. Such hole may be predrilled before insertion of the suture anchor 14, or such hole may be formed by distal advancement of the eyelet 18 and/or the anchor body 16 by the insertion tool 12. The central cannulation of the anchor body 16 extends along the longitudinal axis L of the suture anchor 14 and the insertion tool 12, and it defines a passage 34 having an inner profile along a plane transverse to the longitudinal axis L. That inner profile is complementary to an abutting outer profile of a portion of the insertion tool 12, as discussed below. The complementary profiles may have a variety of shapes, such that the insertion tool 12 can transmit rotational motion to the anchor body 16 about the longitudinal axis L. For example, the profiles may be generally square, as shown in the figures, but many other shaped profiles would be suitable as well, such as hex, star, triangle, etc. The anchor body 16 may also include one or more transverse passages or holes 36 that communicate between the passage 34 and the exterior of the anchor body (*e.g.,* between adjacent turns of a screw thread 32). Such passages may be used, for example, to allow a bioactive material subsequently dispensed into the passage 34 to flow out into contact with the surrounding bone.

The eyelet 18 may have a cannulation along its length that communicates with a distal opening 38. The eyelet 18 may also include one or more lateral openings 40, such as a pair of lateral openings 40 on opposing sides of the eyelet 18 towards its proximal end.

The anchor body 16 and the eyelet 18 may be formed of any material, such as any biocompatible material, and the anchor body 16 and eyelet 18 may be formed of the same material or different materials. For example, either or both of the anchor body 16 and eyelet 18 may be formed of polymeric materials (*e.g*., PEEK, biodegradable materials, etc.), metals (*e.g.,* stainless steel, titanium, etc.), ceramics, tissue-based materials (*e.g.,* allografts, autografts, artificial tissue grafts, etc.), or the like, or any combinations of such materials. In one example, either or both of the anchor body 16 and the eyelet 18 may be formed entirely of PEEK (polyether ether ketone), which is beneficially radiolucent. Moreover, a variety of sizes may be used, as may be appropriate to the relevant anatomy and/or the particular procedure being performed. For example, representative suture anchor bodies 16 may have a diameter orthogonal to the longitudinal axis L in a range from 3.9 mm to 6.5 mm.

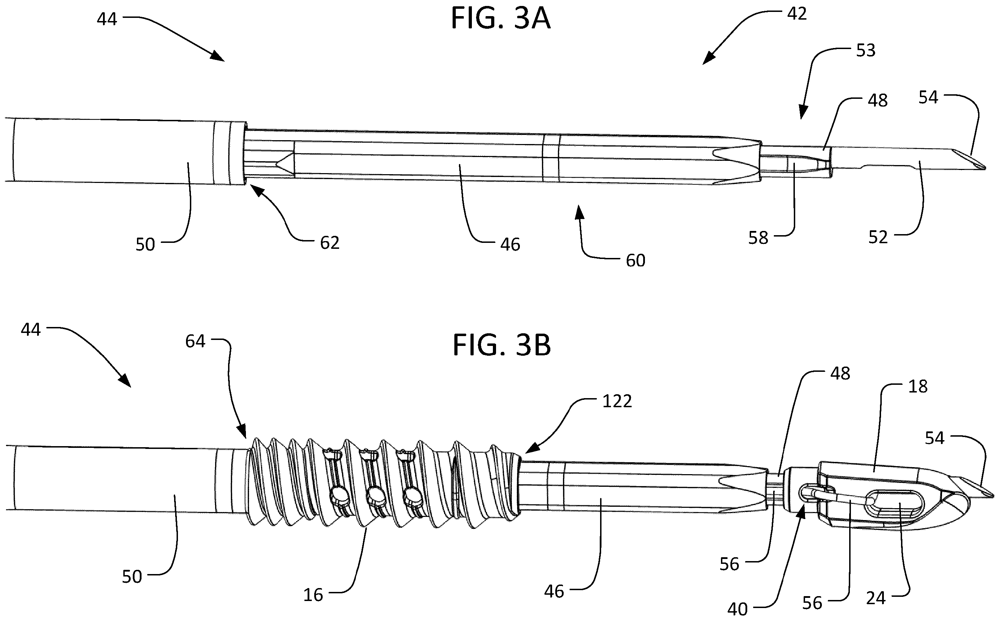

A distal region 42 of an elongated shaft 44 extending distally from the handle 30 of the insertion tool 12 is illustrated in FIG. 3A. The elongated shaft 44 is comprised of three concentric shafts: an intermediate shaft 46, an inner shaft 48 concentrically disposed within the intermediate shaft 46, and an outer shaft 50 concentrically disposed about the intermediate shaft 46. All three shafts are generally cylindrical and elongated along the longitudinal axis L, with at least the intermediate shaft 46 and the outer shaft 50 being cannulated along their length. The inner shaft 48 includes a distally-extending extension 52 at its distal end 53 that is configured to be received within the cannulation of the eyelet 18. The extension 52 may function as structural support for the eyelet 18. The extension may also function as an awl, and thus such extension 52 may include a distal tip 54 configured to project distally outward from the distal opening 38 (shown in FIG. 2) of the eyelet 18. The distal tip 54 may be tapered or otherwise sharpened so that it can help to initiate the formation of a hole in the bone, such as a hole that the eyelet 18 and anchor body 16 may subsequently enlarge upon distal advancement. The inner shaft 48 may also include at least one passageway defined therealong within the intermediate shaft 46 so as to receive one or more free ends of a retention suture 56 therein. The passageway may include multiple passageways, such as a pair of opposing elongated grooves 58 formed along an outer profile of the inner shaft 48. As shown in FIG. 3B, the retention suture 56 helps to secure the eyelet 18 to the distal end of the elongated shaft 44 of the insertion tool 12, and specifically to the distal extension 52 of the inner shaft 48. In particular, the retention suture 56 extends transversely through the opening 24 in the eyelet 18, where opposing free ends of the retention suture 56 pass inwardly through the opposing lateral openings 40 of the eyelet 18 and into the grooves 58 of the inner shaft 48, where the free ends of the retention suture 56 then extend proximally along the grooves 58 encircled by the intermediate shaft 46 up to the handle 30, as discussed in more detail below.

The anchor body 16 is positioned on a distal region 60 of the intermediate shaft 46 such that the intermediate shaft 46 extends through the passage 34 of the anchor body 16. An outer profile of the distal region 60 of the intermediate shaft 46 is complementary to the abutting inner profile of the passage 34 (shown in FIG. 2) of the anchor body 16. Specifically, the passage 34 is keyed to the distal region 60 of the intermediate shaft 46, such that rotation of the intermediate shaft 46 about the longitudinal axis L relative to the inner shaft 48 can rotate the anchor body 16 about the longitudinal axis L so as to helically advance the thread(s) 32 along the bone hole.

The outer shaft 50 has a distal end 62 configured to abut a proximal end 64 of the anchor body 16. Accordingly, by distal advancement of the outer shaft 50 about and relative to the intermediate shaft 46, while the intermediate shaft 46 rotates relative to the inner shaft 48, the insertion tool 12 can cause the anchor body 16 to distally advance within the bone hole towards the eyelet 18 while the thread(s) 32 of the anchor body 16 anchor into the periphery of the bone hole, as discussed in more detail below.

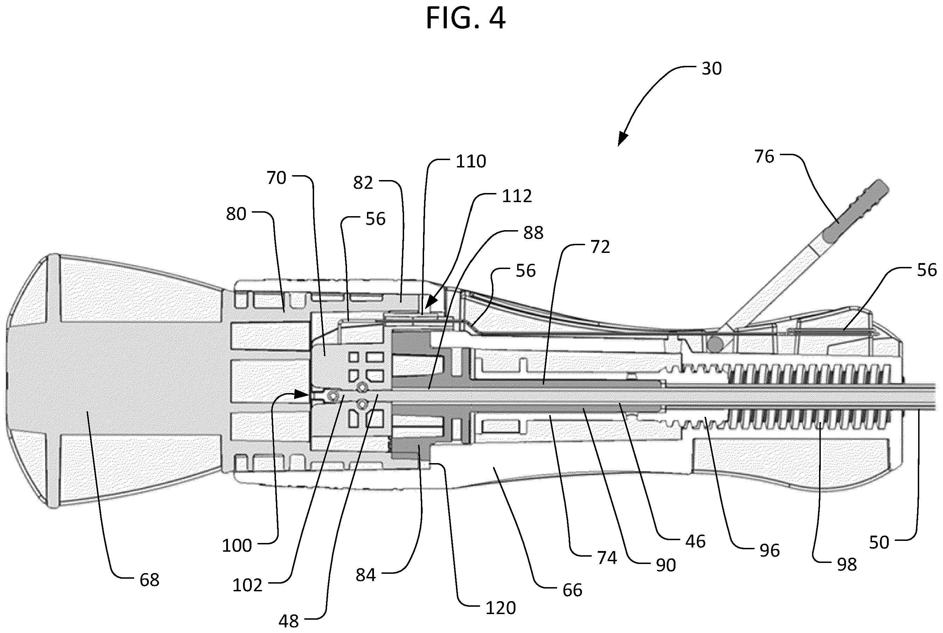

FIG. 4 illustrates a cross sectional view of the handle 30 of the insertion tool 12 taken along the longitudinal axis L (shown in FIG. 1). The handle 30 includes a handle body 66, a knob 68, a suture guide 70, a drive shaft 72, a lead screw member 74, and a lock 76 for securing the free ends of the retention suture 56 with respect to the handle body 66. The components of the handle 30 are connected to the inner shaft 48, the intermediate shaft 46, and the outer shaft 50 such that the inner shaft 48 (and thus the eyelet 18 secured to its distal end 53) is rotationally fixed with respect to the handle body 66, whereas the intermediate shaft 46 and the outer shaft 50 are movable relative to the handle body 66 and the inner shaft 48 so as to helically advance the anchor body 16 distally towards the eyelet 18 and into the bone hole. Such movement of the intermediate shaft 46 and the outer shaft 50 relative to the handle body 66 is driven by rotation of the knob 68 relative to the handle body 66 about the longitudinal axis L of the insertion tool 12, as will now be discussed.

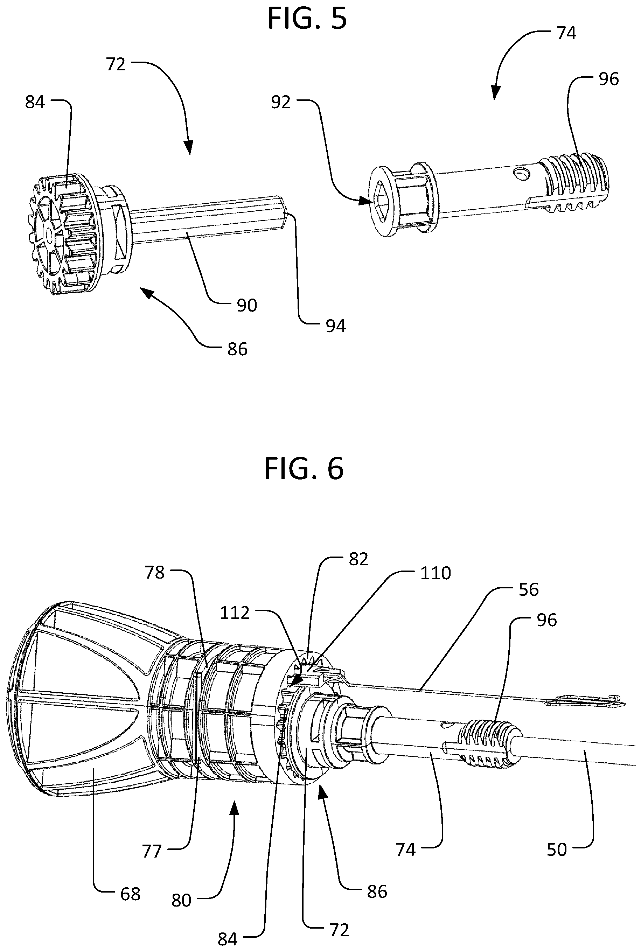

The knob 68 is rotatable relative to the handle body 66 while being secured to the handle body 66 by one or more pins 77 (*e.g.,* spring pins or solid pins) fastened to the handle body 66. As shown in FIG. 6, the pins 77 are tangentially received within a channel 78 extending circumferentially around a distal portion 80 of the knob 68, the distal portion 80 being received within a proximal end of the handle body 66. The distal portion 80 of the knob also includes a ring gear 82 for driving rotation of a spur gear 84 that is connected to the proximal end 86 of the drive shaft 72. The drive shaft 72 is rotationally fixed to the proximal end 88 of the intermediate shaft 46 so that rotation of the drive shaft 72 drives rotation of the intermediate shaft 46 (so as to rotate the anchor body 16 about the longitudinal axis L). For example, the spur gear 84 and drive shaft 72 may be monolithically formed as part of the same piece of material (*e.g.*, plastic) and overmolded onto the intermediate shaft 46. By including (*e.g*., machining) one or more flats on the intermediate shaft 46, the molded spur gear 84 and drive shaft 72 will become rotationally constrained with respect to the intermediate shaft 46. The drive shaft 72 also includes a shaft portion 90 extending distally from the spur gear 84. The shaft portion 90 is configured to transfer torque to the lead screw member 74 while allowing relative translational movement between the drive shaft 72 and the lead screw member 74 along the longitudinal axis L. Accordingly, the shaft portion 90 may be provided with an outer profile that is complementary to an abutting inner profile of a passage 92 (shown in FIG. 5) in the lead screw member 74 within which the shaft portion 90 is configured to be received. For example, the outer profile of the shaft portion 90 along a plane transverse to the longitudinal axis L may have a generally square shape, such that each of the four corners of the square shape effectively defines a spline 94 for transferring torque to the complementarily shaped passage 92 in the lead screw member 74. Moreover, the tolerances are such between the outer profile of the shaft portion 90 and the inner profile of the passage 92 that the lead screw member 74 can translate distally along the longitudinal axis L relative to the drive shaft 72 while the drive shaft 72 induces rotation of the lead screw member 74 about the longitudinal axis L.

The lead screw member 74 includes a threaded region 96 along its outer surface towards the distal end of the lead screw member 74. The threaded region 96 is configured to interface with complementary threads 98 formed on an interior surface of the handle body 66 of the handle 30. That threaded connection is configured such that, upon rotation of the lead screw member 74 induced by rotation of the drive shaft 72, the lead screw member 74 will advance distally along the threads 98 relative to the drive shaft 72 and the handle body 66. The lead screw member 74 is affixed to the outer shaft 50, for example in a similar manner to the connection of the intermediate shaft 46 to the drive shaft 72 and spur gear 84. That is, the lead screw member 74 may be monolithically formed as a single piece of material (*e.g*., plastic) and overmolded onto the outer shaft 50. By including one or more surface features on the outer shaft 50, the lead screw member 74 may become rotationally constrained with respect to the outer shaft 50. Such surface feature may include a knurl or knurling, for example. As a result of such connection between the screw member 74 and the outer shaft 50. Distal advancement of the lead screw member 74 along the threads 98 will correspond to distal advancement of the outer shaft 50 with respect to the intermediate shaft 46 encircled thereby. For example, distal translation and rotation of the lead screw member 74 as it advances along the threads 98 may correspond to distal translation and rotation of the outer shaft 50. As a result of the distal movement of the outer shaft 50 relative to the intermediate shaft 46, the distal end 62 of the outer shaft 50 will push the proximal end 64 of the anchor body 16 distally along the distal region 60 of the intermediate shaft 46 towards the eyelet 18. Moreover, the engagement between the threads 98 of the handle body 66 and the threaded region 96 of the lead screw member 74 is desirably configured to correspond to the profile of the screw threads 32 of the anchor body 16, so that the distal end 62 of the outer shaft 50 pushes the anchor body 16 distally at a rate corresponding to the threaded advancement of the anchor body 16 within the bone hole. For example, the thread pitch of the threaded region 96 of the lead screw member 74 and/or the threads 98 of the handle body 66 desirably match the thread pitch of the threads 32 of the anchor body 16.

In the above-described embodiment, the ring gear 82 has a larger diameter than the diameter of the spur gear 84 received within it, and thus the spur gear is offset to one side of the rotational center of the ring gear 82 so that the teeth of the spur gear 84 mesh with the teeth of the ring gear 82. Due to the size difference between the ring gear 82 (driven by the knob 68) and the spur gear 84 (which is coupled to and drives the intermediate shaft 46 and the outer shaft 50, which in turn drive the helical advancement of the anchor body 16), a gear ratio results between the turning of the knob 68 and the turning of the anchor body 16. Specifically, since the spur gear 84 is smaller than the ring gear 82, the result is that one full rotation of the knob 68 results in at least slightly more than one full rotation (e.g., about 1.2 rotations) of the anchor body 16.

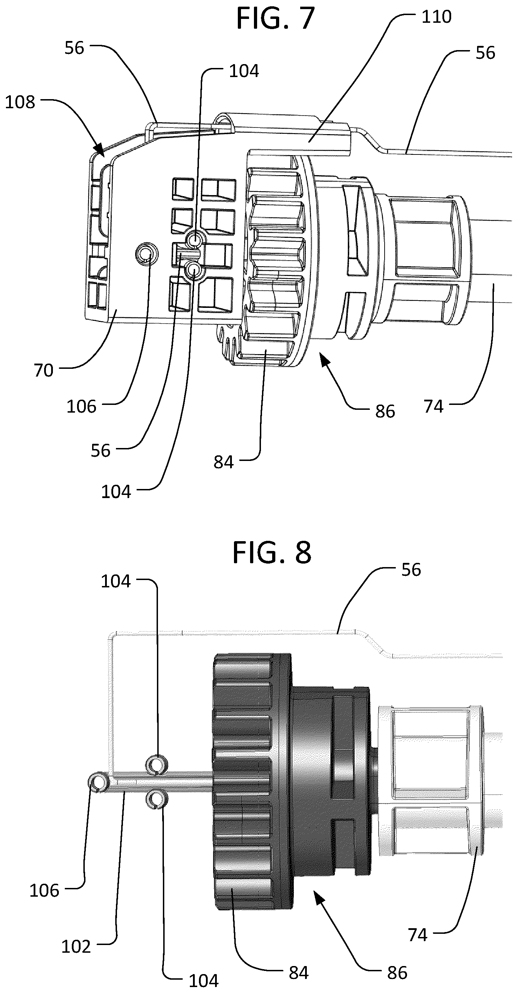

The handle 30 is designed to allow the retention suture 56 to be secured to it so as to help maintain the eyelet 18 in a stable position at the distal end 53 of the inner shaft 48. In that regard, as discussed above, the free ends of the retention suture 56 are seated within respective elongated grooves 58 formed along the inner shaft 48 such that the free ends of the retention suture 56 extend longitudinally in a proximal direction along the inside of the intermediate shaft 46. As shown in FIG. 4, the proximal end 88 of the intermediate shaft 46 terminates within the drive shaft 72. Accordingly, the free ends of the retention suture 56 pass from within the intermediate shaft 46 into the suture guide 70. The suture guide 70 includes a passageway 100 within which the proximal end 102 of the inner shaft 48 is secured. The free ends of the retention sutures 56 pass within the passageway 100. The proximal end 102 of the inner shaft 48 may be secured to the suture guide 70 by a press fit within the passageway 100 and/or by means of one or more pins 104, 106 (*e.g.,* spring pins), as shown in FIGS. 7 and 8. Specifically, the proximal end 102 of the inner shaft 48 may include one or more grooves along its outer surface configured to tangentially receive one or more respective pins 104 that are fixed with respect to the suture guide 70 by being received within one or more respective boreholes in the suture guide 70. Such pins 104 desirably help to fix the longitudinal position of the inner shaft 48 with respect to the suture guide 70. One or more additional pins 106 may also be provided within respective boreholes in the suture guide 70. Specifically, as shown in FIG. 8, a pin 106 may extend transversely with respect to the proximal extremity of the proximal end 102 of the inner shaft 48, such that the pin 106 is at least partially received within a furrow defined in the proximal extremity. As a result of that engagement between the pin 106 and the furrow, the inner shaft 48 is rotationally constrained with respect to the suture guide 70 about the longitudinal axis L.

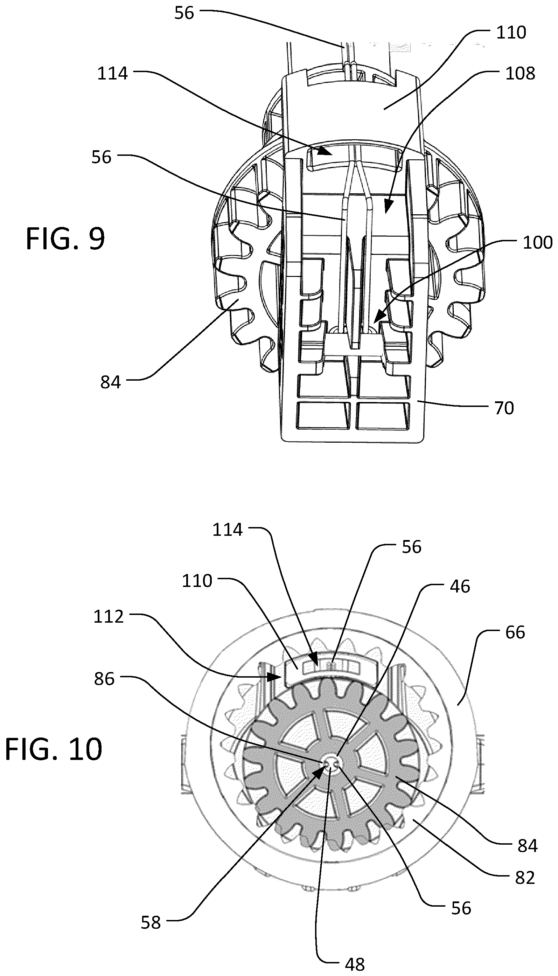

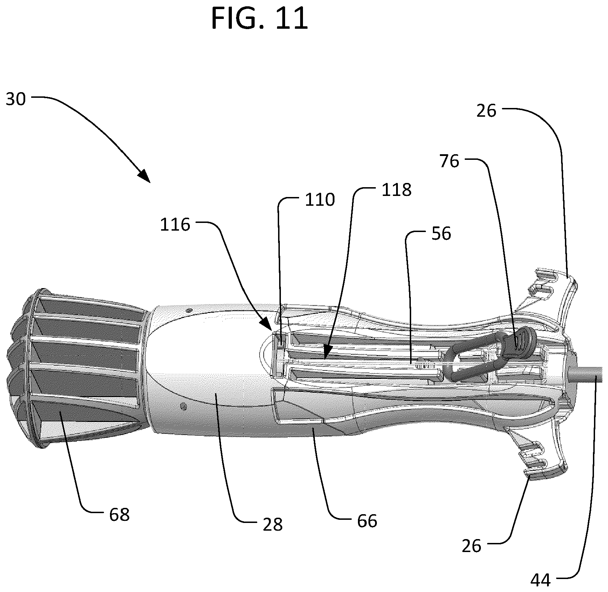

As shown in FIG. 9, the suture guide 70 includes a hollow region 108 within which the free ends of the retention suture 56 are redirected from extending along the longitudinal direction of the elongated shaft 44 so that the free ends of the retention suture 56 can exit the handle 30 of the insertion tool 12. Specifically, the free ends of the retention suture 56 pass proximally out of the passageway 100 of the suture guide 70, where they then pass laterally within the hollow region 108 of the suture guide 70 to a guiding extension 110 that directs the free ends of the retention suture 56 back in the longitudinal direction towards the distal end of the insertion tool 12. The guiding extension 110 is positioned within a space 112 defined between the ring gear 82 and the spur gear 84, as shown in FIGS. 4, 6, and 10. The space 112 is defined by the smaller sizing of the spur gear 84 and the offset of the spur gear 84 from the rotational center of the ring gear 82. The guiding extension 110 preferably encircles an internal passageway 114 defined therein through which the free ends of the retention suture 56 extend, so that the retention suture 56 does not come into contact with the teeth of the ring gear 82 or spur gear 84. As shown in FIG. 11, the guiding extension 110 is aligned with an opening 116 through the handle body 66 so that the retention suture 56 can pass out of the handle 30 through the opening 116. One or more grooves 118 may be defined in the outer surface 28 of the handle body 66 in proximity to the opening 116 so as to receive the free ends of the retention suture 56, which may be secured to the handle body 66 by the lock 76. Specifically, the lock 76 may be pivotably coupled to the handle body 66 along the outer surface 28, such that the lock 76 can be pivoted to a locked position in which the free ends of the retention suture 56 are compressed between the lock 76 and the outer surface 28 of the handle body 66 so as to secure the free ends of the retention suture 56 to the handle body 66.

During the rotational and distal advancement of the anchor body 16 by means of the movement of the intermediate shaft 46 and outer shaft 50 driven by the knob 68 of the handle 30, the eyelet 18 is desirably maintained stationary with respect to the handle body 66. Specifically, the eyelet 18 is secured to the distal end 53 of the inner shaft 48, and the inner shaft is, in turn, secured to the suture guide 70 as described above. The longitudinal position of the suture guide 70 within the handle body 66 may be constrained by the positioning of the suture guide 70 between the knob 68 at its proximal end and the drive shaft 72 at its distal end. The longitudinal position of the drive shaft 72 may, in turn, be constrained in the longitudinal direction by interaction with one or more features on the inside of the handle body 66. For example, one or more ledges 120 (shown in FIG. 4) may be positioned within the inside of the handle body 66 to prevent the drive shaft 72 from being displaced in the distal direction. Rotational orientation of the suture guide 70 may also be constrained by interaction with a portion of the handle body 66. In particular, one or more portions of the handle body 66 adjacent to the opening 116 (such as a slot defined within the handle body 66 that leads to the opening 116) may receive, engage, or otherwise constrain the position of the guiding extension 110 with respect to the handle body 66 so that the rotational orientation of the suture guide 70 about the longitudinal axis L of the insertion tool 12 is fixed with respect to the handle body 66.

Beneficially, by maintaining the eyelet 18 stationary with respect to the handle body 66, including having the rotational orientation of the eyelet 18 fixed with respect to the handle body 66, the retention suture 56 may avoid being twisted (and potentially damaged) during rotational insertion of the anchor body 16. In addition, due to the fixed distance between the handle body 66 and the eyelet 18 (when the eyelet 18 is positioned on the distal end 53 of the inner shaft 48), the tension in the retention suture 56 secured by the lock 76 may be maintained. Similarly, the tension may also be maintained in any repair sutures passing through the opening 24 in the eyelet 18 and having their free ends extending proximally and secured to the one or more cleats 26, since the cleats 26 have a fixed position on the handle body 66.

A method of inserting the suture anchor 14 into bone using the insertion tool 12 disclosed above will now be discussed. It is noted that at least some of the steps may be performed before a user (*e.g*., surgeon) uses the insertion tool 12 to implant the suture anchor 14 into a patient's body. For example, some or all preparatory steps in assembling components of the system may be performed by other medical staff assisting the surgeon. Additionally, or alternatively, some or all preparatory steps may be performed by the manufacturer of the insertion tool 12 and other system components before they are delivered to the user. As an example, the components of the system may be provided by the manufacturer in a kit substantially ready to use, such as in the state shown in FIG. 1. As shown in that figure, that state includes the anchor body 16 and eyelet 18 of the suture anchor 14 being coupled to the insertion tool 12 and at least the retention suture 56 already being fed through the opening 24 of the eyelet 18. Moreover, the free ends of the retention suture 56 extend proximally along the grooves 58 of the inner shaft 48 positioned inside the intermediate shaft 46 and then out of the opening 116 in the handle 30.

Once the suture anchor system 10 is positioned in a configuration like that shown in FIG. 1, one or more repair sutures may be fed through the opening 24 in the eyelet 18, *e.g.,* with the help of the pull tab 20 and associated loop 22 of wire. That is, the repair sutures may be positioned so as to extend through the loop 22, after which the pull tab 20 may be pulled away from the eyelet 18 transverse to the longitudinal axis L, so that the loop 22 and any repair sutures extending through it are pulled through the opening 24 of the eyelet 18. Once the one or more repair sutures are positioned to extend through the opening 24 of the eyelet 18, their free ends may be pulled proximally and secured to one or more of the cleats 26 so as to provide a desired amount of tension in the repair sutures. One or more of the repair sutures may also be secured to the cleats 26 in a slack arrangement (*i.e.,* without any tension in the repair suture(s)), in which case the securement to the cleats 26 may desirably prevent excess length of repair suture being positioned distally of the cleats 26, where it could interfere with or obscure the insertion of the suture anchor or other steps of the procedure.

Once the repair sutures are secured to the cleats 26 and the retention suture 56 is secured to the handle 30 by the lock 76, the handle 30 can be grasped in the hand of the user (*e.g*., surgeon or other medical professional) and advanced with the distal region 42 of the elongated shaft 44 oriented distally towards a target area of bone of a patient. The insertion tool 12 may then be advanced so that the eyelet 18 advances into a hole in the bone. Such bone hole may either have been formed in an earlier step (*e.g*., with an awl, bone drill, or other tool), or the advancement of the eyelet 18 positioned on the distal end 53 of the inner shaft 48 may result in the formation of the bone hole that the eyelet 18 advances into. In that regard, the tapered or sharpened distal tip 54 of the extension 52 that projects distally out of the distal opening 38 of the eyelet 18 may help to form such bone hole. After the eyelet 18 is completely underneath the surface of the bone, continued distal advancement of the insertion tool 12 will bring the distal end 122 (shown in FIGS. 2 and 3B) of the anchor body 16 to the entrance of the bone hole. In such position, the one or more repair sutures will be positioned so as to extend from the opening 24 of the eyelet 18 proximally along the length of the bone hole and then along the outer surface of the anchor body 16.

Next, the anchor body 16 is advanced distally into the bone hole. To accomplish that, the knob 68 is rotated (*e.g*., with one of the user's hands) while relatively firm distal pressure is applied to the insertion tool 12 (*e.g.,* by the other user's hand grasping the handle body 66). The distal pressure on the insertion tool 12 is desirably applied so as to maintain the position of the eyelet 18, the inner shaft 48, and the handle body 66 with respect to the bone hole, while also providing a distal force on the anchor body 16 so that its threads 32 can helically tap their way into the periphery of the bone hole to secure the anchor body 16 within the bone hole. As discussed above, the helical advancement of the anchor body 16 is driven by the rotation of the knob 68. Specifically, rotation of the knob 68 causes rotation of the ring gear 82 coupled to the distal portion 80 of the knob 68. The ring gear 82 engages and drives the spur gear 84 of the drive shaft 72. Such rotation of the drive shaft 72 thus directly rotates the intermediate shaft 46 about the longitudinal axis, which results in rotation of the anchor body 16 about the longitudinal axis due to the keyed relationship between the distal region 60 of the intermediate shaft 46 and the passage 34 within the anchor body 16. Moreover, distal advancement of the anchor body 16 is aided by the distal translation (and rotation) of the outer shaft 50 about and relative to the intermediate shaft 46 due to the distal threaded advancement of the lead screw member 74 along the threads 98 of the handle body 66, since the lead screw member 74 is directly coupled to the outer shaft 50. As discussed above, the distal threaded advancement of the lead screw member 74 is driven by the rotational engagement of the drive shaft 72 (including one or more splines 94 along its outer surface) with the complementarily shaped passage 92 in the lead screw member 74.

As the anchor body 16 advances distally within the bone hole, the strands of the repair suture extending alongside the anchor body 16 become pinched between the anchor body 16 and the periphery of the bone hole so as to securely anchor the repair sutures with respect to the bone. Moreover, although the primary purpose of the retention suture 56 is to help secure the eyelet 18 to the distal end 53 of the inner shaft 48 of the insertion tool 12 during implantation, the retention suture 56 may subsequently be used as a repair suture.

Beneficially, during the helical advancement of the anchor body 16 into the bone hole and distally towards the eyelet 18, the eyelet 18 does not rotate. That is, due to the translational and rotational fixing of the inner shaft 48 (to which the eyelet 18 is secured) relative to the handle body 66, as discussed above, the eyelet 18 is maintained in the same distal position and rotational orientation about the longitudinal axis L as the handle body 66. As a result, the retention suture 56 desirably avoids being twisted and the tension in the retention suture 56 is maintained during rotational advancement of the anchor body 16. In addition, due at least to the distal advancement of the outer shaft 50 relative to the intermediate shaft 46 and the handle body 66, the position of the handle 30 does not have to advance distally along with the anchor body 16 in order to drive the anchor body 16 into the bone hole. Therefore, the handle 30 can maintain a fixed distance from the eyelet 18 during the advancement of the anchor body 16, such that the tension in the repair sutures can be maintained as a result of the fixed positions of the cleats 26 on the stationary handle body 66. Finally, during the advancement of the anchor body 16, the rotation of the knob 68 and the associated internal components of the handle 30 that drive the intermediate shaft 46 and outer shaft do not interfere with the retention suture 56, which passes through the space 112 between the ring gear 82 and spur gear 84, as discussed above, so as to be secured to the outside of the handle body 66 at a fixed tension.

Various alternative embodiments may be possible within the scope of the present invention. For example, it is not necessary that the ring gear 82 be a part of the knob 68 and that the spur gear 84 be a part of the drive shaft 72. In one alternative embodiment, those components can be reversed, such that the spur gear is a distal component of the rotatable knob, whereas the ring gear is a proximal component of the drive shaft. In such alternative embodiment, the free ends of the retention suture would instead extend through the space between the ring and spur gears in the proximal direction, rather than the distal direction as shown in the above embodiment. Once passing in the proximal direction, however, the path of the free ends of the retention suture may be redirected as desired so that they can be transmitted through an opening to the exterior of the handle of the insertion tool. Moreover, by reversing the ring and spur gears, the gear ratio discussed above would also be reversed. That is, a full turn of the rotatable knob would instead correspond to at least slightly less than a full turn (*e.g*., about 0.8 or 0.83̅ rotations) of the anchor body.

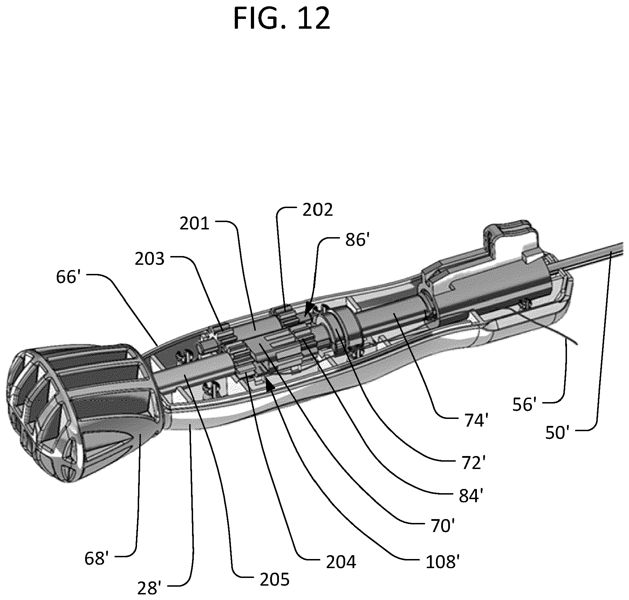

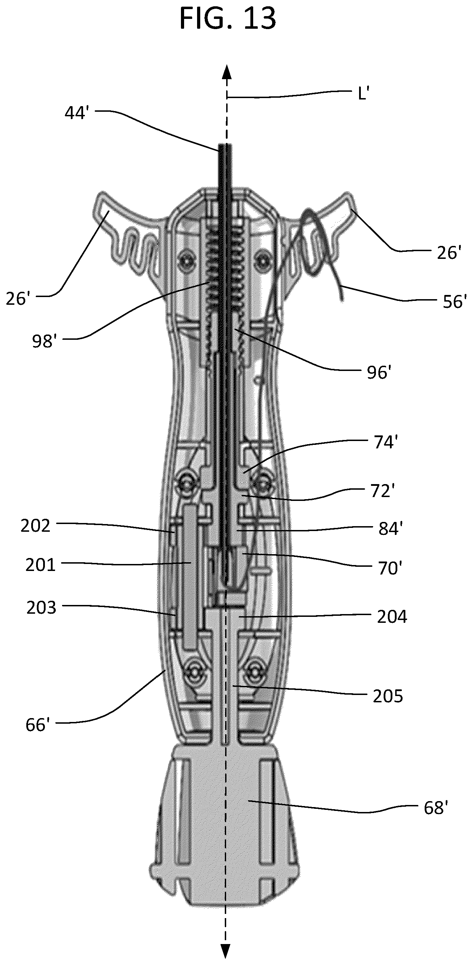

Another alternative embodiment for providing a path for the free ends of the retention suture so that they do not create interference with the rotational components of the insertion tool is shown in FIGS. 12 and 13. In such figures, reference numerals similar to those used previously (except bearing a prime symbol) are used to indicate components that are analogous to those previously-used reference numerals. In view of such similarities to components already discussed herein, a separate discussion is not needed and, for brevity, may not be provided with respect to some or all of the analogous components shown in the embodiment of FIGS. 12 and 13. In such cases, it is to be understood that the discussion of the analogous components of the previous embodiments applies equally to the relevant components of the embodiment of FIGS. 12 and 13. However, new components in the embodiment of FIGS. 12 and 13 that are not analogous to components previously discussed are given reference numerals in the 200 range.

Most notably, in the embodiment of FIGS. 12 and 13, rather than having the torque from the knob transmitted via a ring gear and spur gear, the torque is transmitted in the embodiment of FIGS. 12 and 13 via a laterally-offset shaft 201. Specifically, the proximal end 86' of the drive shaft 72' includes a spur gear 84'. However, rather than engaging a ring gear, the spur gear 84' engages and is driven by another spur gear 202 positioned at the distal end of a shaft 201 that extends along the longitudinal direction but is laterally offset from the longitudinal axis L'. At the proximal end of the shaft 201 is another spur gear 203 that is engaged with a spur gear 204 connected to the rotatable knob 68' via a distally extending shaft 205. A suture guide 70' similar to suture guide 70 described above in connection with the first embodiment is positioned between the spur gear 84' of the drive shaft 72' and the spur gear 204 of the shaft 205 of the rotatable knob 68'. Like the suture guide 70, the suture guide 70' of the embodiment of FIG. 12 and 13 allows the free ends of the retention suture 56' to pass out of the intermediate shaft and then be guided laterally away from the shaft 201 before eventually passing out of an opening in the handle body 66' so that the retention suture 56' can be secured along an outer surface 28' of the handle body 66'. For example, as shown in FIG. 13, the retention suture 56' may be secured to one of the cleats 26' projecting from the outer surface 28'.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. An insertion tool for inserting a suture anchor into bone, comprising:
a handle;
an intermediate shaft extending along a longitudinal axis between a proximal end and a distal end, the proximal end of the intermediate shaft being rotatably coupled to the handle and the distal end of the intermediate shaft being configured to couple to an anchor body of a suture anchor, the intermediate shaft being rotatable relative to the handle so as to rotate the anchor body relative to the handle;
an inner shaft concentrically disposed within the intermediate shaft, the inner shaft having a distal end configured to couple to an eyelet positionable distally of and separate from the anchor body; and
an outer shaft concentrically disposed about the intermediate shaft and configured to translate distally relative to the handle so as to advance the anchor body distally towards the eyelet.

2. The insertion tool of claim 1, wherein the inner shaft is rotationally fixed relative to the handle.

3. The insertion tool of claim 1 or 2, wherein the distal end of the intermediate shaft has a keyed outer profile complementary to an inner profile of a passage within the anchor body.

4. The insertion tool of claim 1, 2 or 3, wherein the handle has a rotatable knob coupled thereto.

5. The insertion tool of claim 4, wherein rotation of the intermediate shaft relative to the handle and distal translation of the outer shaft relative to the handle are both driven by the rotatable knob.

6. The insertion tool of claim 5, wherein a proximal end of the outer shaft is movably coupled to the handle and to the rotatable knob via at least one spline aligned along the longitudinal axis and via a lead screw thread, such that rotation of the rotatable knob drives distal translation of the outer shaft relative to the handle.

7. The insertion tool of any of claims 4-6, wherein the rotatable knob drives rotation of the intermediate shaft relative to the handle via a ring gear engaged with a spur gear, the ring gear and the spur gear defining a space therebetween such that a retention suture coupled to the eyelet and extending within the intermediate shaft can pass through the space and out of the handle.

8. The insertion tool of claim 7, wherein the ring gear and the spur gear define a gear ratio therebetween such that the anchor body rotates at a different rate than the rotatable knob.

9. The insertion tool of any of claims 1-8, wherein the handle includes at least one cleat along an outer surface of the handle, the cleat being configured to secure a free end of a repair suture extending proximally from the eyelet.

10. A system for inserting a suture anchor into bone, comprising:
the insertion tool of any of claims 1-9;
the anchor body; and
the eyelet.

11. The system of claim 10, further comprising:
a repair suture for securing tissue and configured to extend through the eyelet; and
a retention suture for helping to secure the eyelet in a position coupled to the distal end of the inner shaft, the retention suture configured to extend within the intermediate shaft of the insertion tool from the eyelet to the handle.

12. The system of claim 10 or 11 wherein the anchor body has a thread along an outer surface thereof.

13. The system of claim 12, wherein the insertion tool is according to claim 6 or any of claims 7-9 as far as dependent from claim 6, wherein a thread pitch of the lead screw thread matches a thread pitch of the thread along the outer surface of the anchor body, such that the outer shaft translates distally relative to the handle to match distal threaded advancement of the anchor body into bone.

14. The system of claim 10-13,
wherein the eyelet is coupled to a distal end of the inner shaft of the insertion tool; and
the system is configured for distally advancing the externally threaded anchor body of the suture anchor into a bore within the bone by rotating the anchor body about the axis while pushing the anchor body distally into the bore along the axis, wherein the rotating of the anchor body about the axis includes rotating the intermediate shaft of the insertion tool about the axis, the intermediate shaft having the distal end coupled to the anchor body, and wherein the pushing of the anchor body distally into the bore includes translating the outer shaft of the insertion tool distally relative to the intermediate shaft.

15. The system of claim 11, or any of claims 12-14 as far as dependent from claim 11, wherein the repair suture extends through the eyelet and has a free end secured to the cleat of the handle of the insertion tool, and wherein the system is configured to maintain tension in the repair suture between the eyelet and the cleat while distally advancing the anchor, such as by maintaining a distance between the handle and the distal end of the inner shaft while the anchor body is distally advanced.
